(19) Europäisches Patentamt
European Patent Office
Office européen des brevets

(11) EP 2 544 689 B1

(12) EUROPEAN PATENT SPECIFICATION

(45) Date of publication and mention
of the grant of the patent:
**11.01.2017 Bulletin 2017/02**

(51) Int Cl.:
*A61K 9/10* *(2006.01)*  *A61K 31/497* *(2006.01)*
*A61K 9/20* *(2006.01)*  *A61K 47/30* *(2006.01)*
*A61P 31/14* *(2006.01)*  *A61K 9/14* *(2006.01)*
*A61K 9/16* *(2006.01)*

(21) Application number: **11753907.2**

(22) Date of filing: **08.03.2011**

(86) International application number:
**PCT/US2011/027511**

(87) International publication number:
**WO 2011/112558 (15.09.2011 Gazette 2011/37)**

(54) **SOLID COMPOSITIONS**

FESTSTOFFZUSAMMENSETZUNGEN

COMPOSITIONS SOLIDES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **10.03.2010 US 339964 P**

(43) Date of publication of application:
**16.01.2013 Bulletin 2013/03**

(73) Proprietor: **AbbVie Bahamas Ltd.**
**New Providence, Nassau (BS)**

(72) Inventors:
• **LIEPOLD, Bernd**
  **29221 Dossenheim (DE)**
• **ROSENBLATT, Karin**
  **68163 Mannheim (DE)**
• **HÖLIG, Peter**
  **63607 Waechtersbach (DE)**
• **GOKHALE, Rajeev**
  **Libertyville**
  **IL 60048 (US)**
• **PRASAD, Leena**
  **Chicago**
  **IL 60608 (US)**
• **MILLER, Jonathan**
  **Lindenhurst**
  **IL 60046 (US)**
• **SCHMITT, Eric, A.**
  **Libertyville**
  **IL 60048 (US)**
• **MORRIS, John, B.**
  **Grayslake**
  **IL 60030 (US)**

(74) Representative: **Modiano, Micaela Nadia et al**
**Modiano & Partners (DE)**
**Thierschstrasse 11**
**80538 München (DE)**

(56) References cited:
**US-A1- 2003 195 228    US-A1- 2004 229 776**
**US-A1- 2006 068 007    US-A1- 2009 269 305**
**US-A1- 2010 144 608    US-A1- 2010 168 384**

EP 2 544 689 B1

**Description**

FIELD OF THE INVENTION

The present invention relates to solid compositions comprising anti-HCV compounds and methods of using the same to treat HCV infection.

BACKGROUND

The hepatitis C virus (HCV) is an RNA virus belonging to the Hepacivirus genus in the Flaviviridae family. The enveloped HCV virion contains a positive stranded RNA genome encoding all known virus-specific proteins in a single, uninterrupted, open reading frame. The open reading frame comprises approximately 9500 nucleotides and encodes a single large polyprotein of about 3000 amino acids. The polyprotein comprises a core protein, envelope proteins E1 and E2, a membrane bound protein p7, and the non-structural proteins NS2, NS3, NS4A, NS4B, NS5A and NS5B.

HCV infection is associated with progressive liver pathology, including cirrhosis and hepatocellular carcinoma. Chronic hepatitis C may be treated with peginterferon-alpha in combination with ribavirin. Substantial limitations to efficacy and tolerability remain as many users suffer from side effects, and viral elimination from the body is often inadequate. Therefore, there is a need for new drugs to treat HCV infection.

SUMMARY OF THE INVENTION

The present disclosure features solid compositions comprising (2R,6S,13aS,14aR,16aS,Z)-N-(cyclopropylsulfonyl)-6-(5-methylpyrazine-2-carboxamido)-5,16-dioxo-2-(phenanthridin-6-yloxy)-1,2,3,5,6,7,8,9,10,11,13a,14,14a,15,16,16a-hexadecahydrocyclopropa[e]pyrrolo[1,2-a][1,4]diazacyclopentadecine-14a-carboxamide (hereinafter Compound I) or a pharmaceutical acceptable salt thereof. Compound I is a potent HCV inhibitor. The solid compositions of the disclosure comprise (1) Compound I (or a pharmaceutically acceptable salt thereof) in an amorphous form, (2) a pharmaceutically acceptable hydrophilic polymer, and (3) a pharmaceutically acceptable surfactant.

In one aspect, the present invention features a solid composition comprising a solid dispersion, wherein the solid dispersion comprises Compound I (or a pharmaceutically acceptable salt thereof) in an amorphous form and a pharmaceutically acceptable hydrophilic polymer, and the solid composition further comprises a pharmaceutically acceptable surfactant. The surfactant can be, without limitation, either formulated in the solid dispersion or separately combined or mixed with the solid dispersion. Preferably, the hydrophilic polymer has a Tg of at least 50 °C. More preferably, the hydrophilic polymer has a Tg of at least 80 °C. Highly preferably, the hydrophilic polymer has a Tg of at least 100 °C. Also preferably, the surfactant has a HLB value of at least 10. Hydrophilic polymers with Tg of at least 25 °C can also be used.

In one embodiment of this aspect of the invention, the hydrophilic polymer is selected from homopolymer of N-vinyl lactam, copolymer of N-vinyl lactam, cellulose ester, cellulose ether, polyalkylene oxide, polyacrylate, polymethacrylate, polyacrylamide, polyvinyl alcohol, vinyl acetate polymer, oligosaccharide, or polysaccharide. Non-limiting examples of suitable hydrophilic polymers include homopolymer of N-vinyl pyrrolidone, copolymer of N-vinyl pyrrolidone, copolymer of N-vinyl pyrrolidone and vinyl acetate, copolymer of N-vinyl pyrrolidone and vinyl propionate, polyvinylpyrrolidone, methylcellulose, ethylcellulose, hydroxyalkylcelluloses, hydroxypropylcellulose, hydroxyalkylalkylcellulose, hydroxypropylmethylcellulose, cellulose phthalate, cellulose succinate, cellulose acetate phthalate, hydroxypropylmethylcellulose phthalate, hydroxypropylmethylcellulose succinate, hydroxypropylmethylcellulose acetate succinate, polyethylene oxide, polypropylene oxide, copolymer of ethylene oxide and propylene oxide, methacrylic acid/ethyl acrylate copolymer, methacrylic acid/methyl methacrylate copolymer, butyl methacrylate/2-dimethylaminoethyl methacrylate copolymer, poly(hydroxyalkyl acrylate), poly(hydroxyalkyl methacrylate), copolymer of vinyl acetate and crotonic acid, partially hydrolyzed polyvinyl acetate, carrageenan, galactomannan, or xanthan gum.

In another embodiment of this aspect of the invention, the surfactant is selected from polyoxyethylene castor oil derivates, mono fatty acid ester of polyoxyethylene sorbitan, polyoxyethylene alkyl ether, polyoxyethylene alkylaryl ether, polyethylene glycol fatty acid ester, alkylene glycol fatty acid mono ester, sucrose fatty acid ester, or sorbitan fatty acid mono ester. Non-limiting examples of suitable surfactants include polyoxyethyleneglycerol triricinoleate or polyoxyl 35 castor oil (Cremophor® EL; BASF Corp.) or polyoxyethyleneglycerol oxystearate such as polyethylenglycol 40 hydrogenated castor oil (Cremophor® RH 40, also known as polyoxyl 40 hydrogenated castor oil or macrogolglycerol hydroxystearate) or polyethylenglycol 60 hydrogenated castor oil (Cremophor® RH 60), mono fatty acid ester of polyoxyethylene sorbitan, such as mono fatty acid ester of polyoxyethylene (20) sorbitan, e.g. polyoxyethylene (20) sorbitan monooleate (Tween® 80), polyoxyethylene (20) sorbitan monostearate (Tween® 60), polyoxyethylene (20) sorbitan monopalmitate (Tween® 40) or polyoxyethylene (20) sorbitan monolaurate (Tween® 20), polyoxyethylene (3) lauryl ether, polyoxyethylene (5) cetyl ether, polyoxyethylene (2) stearyl ether, polyoxyethylene (5) stearyl ether, polyoxyethylene (2) nonylphenyl ether, polyoxyethylene (3) nonylphenyl ether, polyoxyethylene (4) nonylphenyl ether, polyoxyethylene (3) octylphenyl ether, PEG-200 monolaurate, PEG-200 dilaurate, PEG-300 dilaurate, PEG-400 dilaurate, PEG-300 distearate, PEG-300 dioleate, propylene glycol monolaurate, sucrose monostearate, sucrose distearate, sucrose monolaurate, sucrose dilaurate, sorbitan mono laurate, sorbitan monooleate, sorbitan monopalnitate, or sorbitan stearate.

In yet another embodiment, the solid dispersion is an amorphous solid dispersion. In still another embodiment, the solid dispersion is an amorphous solid dispersion which comprises Compound I (or a pharmaceutically acceptable salt thereof), the hydrophilic polymer, and the surfactant. In a further embodiment, the solid dispersion is a solid solution comprising Compound I (or a pharmaceutically acceptable salt thereof) and the hydrophilic polymer. In yet another embodiment, the solid dispersion is a solid solution comprising Compound I (or a pharmaceutically acceptable salt thereof), the hydrophilic polymer and the surfactant.

In yet another embodiment of this aspect of the invention, the hydrophilic polymer is a homopolymer or copolymer of N-vinyl pyrrolidone. Preferably, the hydrophilic polymer is copovidone.

In still another embodiment, the surfactant is propylene glycol laurate (e.g., lauroglycol FCC from Gattefosse). The solid composition may further comprise another pharmaceutically acceptable surfactant such as D-alpha-tocopheryl polyethylene glycol 1000 succinate (vitamin E TPGS).

In still yet another embodiment, the surfactant is a polysorbate. Preferably, the surfactant is polysorbate 80 (Tween 80).

In yet another embodiment, a solid composition of the disclosure comprises an amorphous solid dispersion or a solid solution which comprises Compound I (or a pharmaceutically acceptable salt thereof), copovidone, and a surfactant selected from polysorbate (preferably polysorbate 80), vitamin E TPGS or a combination of vitamin E TPGS and propylene glycol laurate (e.g., lauroglycol FCC).

A solid composition of the present disclosure may further include ritonavir, preferably a solid dispersion of ritonavir. Ritonavir and Compound I (or a pharmaceutically acceptable salt thereof) may be formulated in the same solid dispersion or solid solution; they may be also formulated in different solid dispersions or solid solutions.

In another aspect, the present invention features processes of making a solid composition of the present invention. In one embodiment, the process comprises drying a solvent in a liquid solution, wherein said solution comprises: (1) Compound I or a pharmaceutically acceptable salt thereof; (2) a pharmaceutically acceptable hydrophilic polymer; and optionally (3) a pharmaceutically acceptable surfactant. The drying process can be carried out using any suitable solvent evaporation techniques including but not limited to spray-drying techniques.

In another embodiment, the process comprises solidifying a melt which comprises: (1) Compound I or a pharmaceutically acceptable salt thereof; (2) a pharmaceutically acceptable hydrophilic polymer; and optionally (3) a pharmaceutically acceptable surfactant.

A solid composition of the invention may also contain other additives or ingredients, such as coloring agents, flavoring agents, lubricants or preservatives. A solid composition of the invention can be prepared into any suitable dosage forms, such as capsule, dragee, granule, powder, or tablet.

A solid composition of the invention may further comprise another anti-HCV agent, for example, an agent selected from HCV helicase inhibitors, HCV polymerase inhibitors, HCV protease inhibitors, HCV NS5A inhibitors, CD81 inhibitors, cyclophilin inhibitors, or internal ribosome entry site (IRES) inhibitors.

The present disclosure further features methods of using a solid composition of the present invention to treat HCV infection. The methods comprise administering a solid composition of the present disclosure to a patient in need thereof, thereby reducing the blood or tissue level of HCV virus in the patient.

Other features, objects, and advantages of the present invention are apparent in the detailed description that follows. It should be understood, however, that the detailed description, while indicating preferred embodiments of the invention, are given by way of illustration only, not limitation. Various changes and modifications within the scope of the invention will become apparent to those skilled in the art from the detailed description.

## DETAILED DESCRIPTION

The present disclosure features solid compositions comprising amorphous Compound I (or a pharmaceutically acceptable salt thereof), a pharmaceutically acceptable hydrophilic polymer, and a pharmaceutically acceptable surfactant. Compound I has low aqueous solubility, and its *in vivo* absorption is expected to be dissolution-rate limited. Formulating Compound I in an amorphous form can increase the inherent drug solubility and dissolution rate, thereby enhancing the bioavailability of the compound. A solid composition of the disclosure can also include ritonavir. Ritonavir is a potent inhibitor of cytochrome P450 3A4 enzyme (CYP3A4), and CYP3A4 is believed to be involved in the metabolism of Compound I. Therefore, co-administering Compound I with ritonavir can reduce the metabolism of Compound I, thereby improving the bioavailability of Compound I.

A non-limiting way to form an amorphous form of Compound I or a combination of Compound I and ritonavir is through the formation of solid dispersions with a polymeric carrier. The presence of hydrophilic polymer(s) and surfactant(s), as well as the dispersion of Compound I in an amorphous form in a matrix containing the polymer(s), can significantly enhance the dissolution rate of the poorly soluble Compound I. In many cases, a solid dispersion formulation can also effectively maintain Compound I in its supersaturation state to allow for better absorption.

As used herein, the term "solid dispersion" defines a system in a solid state (as opposed to a liquid or gaseous state) comprising at least two components, wherein one component is dispersed throughout the other component or components. For example, an active ingredient or a combination of active ingredients can be dispersed in a matrix comprised of a pharmaceutically acceptable hydrophilic polymer(s) and a pharmaceutically acceptable surfactant(s). The term "solid dispersion" encompasses systems having small particles of one phase dispersed in another phase. These particles are often of less than 400 $\mu$m in size, such as less than 100, 10, or 1 $\mu$m in size. When a solid dispersion of the components is such that the system is chemically and physically uniform or homogenous throughout or consists of one phase (as defined in thermodynamics), such a solid dispersion is called a "solid solution." A glassy solution is a solid solution in which a solute is dissolved in a glassy solvent.

The term "$AUC_\infty$" refers to the area under the plasma concentration time curve (AUC) extrapolated to infinity.

The terms "weight percent" or "percent by weight" or "% by weight" or "wt %" denote the weight of an individual component in a composition or mixture as a percentage of the weight of the composition or mixture.

In one aspect, the present invention features a solid composition comprising Compound I (or a pharmaceutically acceptable salt thereof) in an amorphous form, a pharmaceutically acceptable hydrophilic polymer, and a pharmaceutically acceptable surfactant. The Compound I (or the salt thereof) and the polymer are formulated in a solid dispersion. The surfactant may also be formulated in the same solid dispersion; or the surfactant can be separately combined or mixed with the solid dispersion.

In one embodiment, a solid composition of the invention comprises an amorphous solid dispersion which comprises Compound I (or a pharmaceutically acceptable salt thereof), a pharmaceutically acceptable hydrophilic polymer and a pharmaceutically acceptable surfactant. In another embodiment, a solid composition of the invention comprises a solid solution which comprises Compound I (or a pharmaceutically acceptable salt thereof) and a pharmaceutically acceptable hydrophilic polymer. In still another embodiment, a solid composition of the invention comprises a solid solution which comprises Compound I (or a pharmaceutically acceptable salt thereof), a pharmaceutically acceptable hydrophilic polymer and a pharmaceutically acceptable surfactant. In yet another embodiment, a solid composition of the invention comprises a glassy solution which includes Compound I (or a pharmaceutically acceptable salt thereof) and a pharmaceutically acceptable hydrophilic polymer. In a further embodiment, a solid composition of the invention comprises a glassy solution which includes Compound I (or a pharmaceutically acceptable salt thereof), a pharmaceutically acceptable hydrophilic polymer and a pharmaceutically acceptable surfactant.

A solid composition of the invention can further comprise a solid dispersion of ritonavir. Preferably, the solid composition comprises a solid solution of ritonavir. More preferably, the solid composition comprises a glassy solution of ritonavir. Compound I (or a pharmaceutically acceptable salt thereof) and ritonavir can be formulated in the same solid dispersion or solid solution. They may also be formulated in separate solid dispersions or solid solutions, which can then be combined or mixed to form a solid composition of the present invention.

In yet another embodiment, a solid composition of the invention comprises an amorphous solid dispersion which includes Compound I (or a pharmaceutically acceptable salt thereof), ritonavir and a pharmaceutically acceptable hydrophilic polymer. In another embodiment, a solid composition of the invention comprises an amorphous solid dispersion which includes Compound I (or a pharmaceutically acceptable salt thereof), ritonavir, a pharmaceutically acceptable hydrophilic polymer and a pharmaceutically acceptable surfactant. In still another embodiment, a solid composition of the invention comprises a solid solution which includes Compound I (or a pharmaceutically acceptable salt thereof), ritonavir and a pharmaceutically acceptable hydrophilic polymer. In still yet another embodiment, a solid composition of the invention comprises a solid solution which includes Compound I (or a pharmaceutically acceptable salt thereof), ritonavir, a pharmaceutically acceptable hydrophilic polymer and a pharmaceutically acceptable surfactant.

In yet another embodiment, a solid composition of the invention comprises a first amorphous solid dispersion which includes Compound I (or a pharmaceutically acceptable salt thereof) and a pharmaceutically acceptable hydrophilic polymer, and a second amorphous solid dispersion comprising ritonavir. In another embodiment, a solid composition of the invention comprises a first amorphous solid dispersion which includes Compound I (or a pharmaceutically acceptable salt thereof), a pharmaceutically acceptable hydrophilic polymer and a pharmaceutically acceptable surfactant, and a second amorphous solid dispersion comprising ritonavir. In still another embodiment, a solid composition of the invention comprises a first solid solution which includes Compound I (or a pharmaceutically acceptable salt thereof) and a pharmaceutically acceptable hydrophilic polymer, and a second solid solution comprising ritonavir. In another embodiment, a solid composition of the invention comprises a first solid solution which includes Compound I (or a pharmaceutically acceptable salt thereof), a pharmaceutically acceptable hydrophilic polymer and a pharmaceutically acceptable surfactant, and a second solid solution comprising ritonavir.

Preferably, a solid dispersion or solid solution that contains ritonavir also includes a pharmaceutically acceptable surfactant to improve the dissolution and/or bioavailability of ritonavir.

The weight ratio of Compound I over ritonavir in a solid composition of the invention may range, without limitation, from 1:1 to 5: 1. Preferably, the weight ratio of Compound I over ritonavir is 2:1, 3:1, or 4:1.

A solid composition of the invention can contain, for example, from 1 to 50% by weight of Compound I. For instance, a solid composition of the invention can contain from 5 to 30% by weight of Compound I. Preferably, a solid composition of the invention contains from 10 to 25% by weight of Compound I.

A solid dispersion of the invention may contain at least 30% by weight of a pharmaceutically acceptable hydrophilic polymer or a combination of such hydrophilic polymers. Preferably, the solid dispersion contains at least 40% by weight of a pharmaceutically acceptable hydrophilic polymer or a combination of such hydrophilic polymers. More preferably, the solid dispersion contains at least 50% (including, e.g., at least 60%, 70% or 80%) by weight of a pharmaceutically acceptable hydrophilic polymer or a combination of such polymers. A solid dispersion of the invention may also contain at least 1% by weight of a pharmaceutically acceptable surfactant or a combination of such surfactants. Preferably, the solid dispersion contains at least 2% by weight of a pharmaceutically acceptable surfactant or a combination of such surfactants. More preferably, the solid dispersion contains from 4% to 20% by weight of the surfactant(s), such as from 5% to 10% by weight of the surfactant(s).

In one embodiment, a solid dispersion of the invention comprises at least 30% by weight of a pharmaceutically acceptable hydrophilic polymer or a combination of such polymers, and at least 1% by weight of a pharmaceutically acceptable surfactant or a combination of such surfactants. In another embodiment, a solid dispersion of the invention comprises at least 50% by weight of a pharmaceutically acceptable hydrophilic polymer or a combination of such polymers, and from 2% to 20% by weight of a pharmaceutically acceptable surfactant or a combination of such surfactants. In yet another embodiment, a solid dispersion of the invention comprises from 50% to 90% by weight of a pharmaceutically acceptable hydrophilic polymer or a combination of such polymers, and from 3% to 15% by weight of a pharmaceutically acceptable surfactant or a combination of such surfactants. In yet another embodiment, a solid dispersion of the invention comprises from 60% to 80% by weight of a pharmaceutically acceptable hydrophilic polymer or a combination of such polymers, and from 5% to 10% by weight of a pharmaceutically acceptable surfactant or a combination of such surfactants.

Preferably, a hydrophilic polymer employed in the present invention has a Tg of at least 50 °C, more preferably at least 60 °C, and highly preferably at least 80 °C including, but not limited to from, 80 °C to 180 °C, or from 100 °C to 150 °C. Methods for determining Tg values of organic polymers are described in INTRODUCTION TO PHYSICAL POLYMER SCIENCE (2nd Edition by L.H. Sperling, published by John Wiley & Sons, Inc., 1992). The Tg value can be calculated as the weighted sum of the Tg values for homopolymers derived from each of the individual monomers, i.e., the polymer Tg = $\sum W_i \cdot X_i$ where $W_i$ is the weight percent of monomer i in the organic polymer, and $X_i$ is the Tg value for the homopolymer derived from monomer i. Tg values for the homopolymers may be taken from POLYMER HANDBOOK (2nd Edition by J. Brandrup and E.H. Immergut, Editors, published by John Wiley & Sons, Inc., 1975). Hydrophilic polymers with a Tg as described above may allow for the preparation of solid dispersions that are mechanically stable and, within ordinary temperature ranges, sufficiently temperature stable so that the solid dispersions may be used as dosage forms without further processing or be compacted to tablets with only a small amount of tabletting aids. Hydrophilic polymers having a Tg of below 50°C may also be used.

Preferably, a hydrophilic polymer employed in the present invention is water-soluble. A solid composition of the present invention can also comprise poorly water-soluble or water-insoluble polymer or polymers, such as cross-linked polymers. A hydrophilic polymer comprised in a solid composition of the present invention preferably has an apparent viscosity, when dissolved at 20 °C in an aqueous solution at 2 % (w/v), of 1 to 5000 mPa·s., and more preferably of 1 to 700 mPa·s, and most preferably of 5 to 100 mPa·s.

Hydrophilic polymers suitable for use in a solid composition of the invention include, but are not limited to, homopolymers or copolymers of N-vinyl lactams, such as homopolymers or copolymers of N-vinyl pyrrolidone (e.g., polyvinylpyrrolidone (PVP), or copolymers of N-vinyl pyrrolidone and vinyl acetate or vinyl propionate); cellulose esters or cellulose ethers, such as alkylcelluloses (e.g., methylcellulose or ethylcellulose), hydroxyalkylcelluloses (e.g., hydroxypropylcellulose), hydroxyalkylalkylcelluloses (e.g., hydroxypropylmethylcellulose), and cellulose phthalates or succinates (e.g., cellulose acetate phthalate and hydroxypropylmethylcellulose phthalate, hydroxypropylmethylcellulose succinate, or hydroxypropylmethylcellulose acetate succinate); high molecular polyalkylene oxides, such as polyethylene oxide, polypropylene oxide, and copolymers of ethylene oxide and propylene oxide; polyacrylates or polymethacrylates, such as methacrylic acid/ethyl acrylate copolymers, methacrylic acid/methyl methacrylate copolymers, butyl methacrylate/2-dimethylaminoethyl methacrylate copolymers, poly(hydroxyalkyl acrylates), and poly(hydroxyalkyl methacrylates); polyacrylamides; vinyl acetate polymers, such as copolymers of vinyl acetate and crotonic acid, and partially hydrolyzed polyvinyl acetate (also referred to as partially saponified "polyvinyl alcohol"); polyvinyl alcohol; oligo- or polysaccharides, such as carrageenans, galactomannans, and xanthan gum; polyhydroxyalkylacrylates; polyhydroxyalkyl-methacrylates; copolymers of methyl methacrylate and acrylic acid; polyethylene glycols (PEGs); or any mixture thereof.

Non-limiting examples of preferred hydrophilic polymers for the invention include polyvinylpyrrolidone (PVP) K17, PVP K25, PVP K30, PVP K90, hydroxypropyl methylcellulose (HPMC) E3, HPMC E5, HPMC E6, HPMC E15, HPMC K3, HPMC A4, HPMC A15, HPMC acetate succinate (AS) LF, HPMC AS MF, HPMC AS HF, HPMC AS LG, HPMC AS MG, HPMC AS HG, HPMC phthalate (P) 50, HPMC P 55, Ethocel 4, Ethocel 7, Ethocel 10, Ethocel 14, Ethocel 20, copovidone (vinylpyrrolidone-vinyl acetate copolymer 60/40), polyvinyl acetate, methacrylate/methacrylic acid copolymer (Eudragit) L100-55, Eudragit L100, Eudragit S100, polyethylene glycol (PEG) 400, PEG 600, PEG 1450, PEG 3350, PEG 4000, PEG 6000, PEG 8000, poloxamer 124, poloxamer 188, poloxamer 237, poloxamer 338, and poloxamer 407.

Of these, homopolymers or copolymers of N-vinyl pyrrolidone, such as copolymers of N-vinyl pyrrolidone and vinyl acetate, are preferred. A non-limiting example of a preferred polymer is a copolymer of 60 % by weight of N-vinyl pyrrolidone and 40 % by weight of vinyl acetate. Other preferred polymers include, without limitation, hydroxypropyl methylcellulose (HPMC, also known as hypromellose in USP), such as hydroxypropyl methylcellulose grade E5 (HPMC-E5); and hydroxypropyl methylcellulose acetate succinate (HPMC-AS).

A pharmaceutically acceptable surfactant employed in the present invention is preferably a non-ionic surfactant. More preferably, a solid composition of the present invention comprises a pharmaceutically acceptable surfactant having an HLB value of at least 10. A solid composition of the present invention can also include a mixture of pharmaceutically acceptable surfactants, with at least one surfactant having an HLB value of no less than 10 and at least another surfactant having an HLB value of below 10. In one example, each surfactant comprised in a solid composition of the invention has an HLB value of at least 10. In another example, each surfactant comprised in a solid composition of the invention has an HLB value of below 10. In yet another example, a solid composition of the present invention includes at least two pharmaceutically acceptable surfactants, one having an HLB value of at least 10 and the other having an HLB value of below 10. The HLB system (Fiedler, H.B., ENCYLOPEDIA OF EXCIPIENTS, 5th ed., Aulendorf: ECV-Editio-Cantor-Verlag (2002)) attributes numeric values to surfactants, with lipophilic substances receiving lower HLB values and hydrophilic substances receiving higher HLB values.

Non-limiting examples of pharmaceutically acceptable surfactants that are suitable for the present invention include polyoxyethylene castor oil derivates, e.g. polyoxyethyleneglycerol triricinoleate or polyoxyl 35 castor oil (Cremophor® EL; BASF Corp.) or polyoxyethyleneglycerol oxystearate such as polyethylenglycol 40 hydrogenated castor oil (Cremophor® RH 40, also known as polyoxyl 40 hydrogenated castor oil or macrogolglycerol hydroxystearate) or polyethylenglycol 60 hydrogenated castor oil (Cremophor® RH 60); or a mono fatty acid ester of polyoxyethylene sorbitan, such as a mono fatty acid ester of polyoxyethylene (20) sorbitan, e.g. polyoxyethylene (20) sorbitan monooleate (Tween® 80), polyoxyethylene (20) sorbitan monostearate (Tween® 60), polyoxyethylene (20) sorbitan monopalmitate (Tween® 40), or polyoxyethylene (20) sorbitan monolaurate (Tween® 20). Other non-limiting examples of suitable surfactants include polyoxyethylene alkyl ethers, e.g. polyoxyethylene (3) lauryl ether, polyoxyethylene (5) cetyl ether, polyoxyethylene (2) stearyl ether, polyoxyethylene (5) stearyl ether; polyoxyethylene alkylaryl ethers, e.g. polyoxyethylene (2) nonylphenyl ether, polyoxyethylene (3) nonylphenyl ether, polyoxyethylene (4) nonylphenyl ether, polyoxyethylene (3) octylphenyl ether; polyethylene glycol fatty acid esters, e.g. PEG-200 monolaurate, PEG-200 dilaurate, PEG-300 dilaurate, PEG-400 dilaurate, PEG-300 distearate, PEG-300 dioleate; alkylene glycol fatty acid mono esters, e.g. propylene glycol monolaurate (Lauroglycol®); sucrose fatty acid esters, e.g. sucrose monostearate, sucrose distearate, sucrose monolaurate, sucrose dilaurate; sorbitan fatty acid mono esters such as sorbitan mono laurate (Span® 20), sorbitan monooleate, sorbitan monopalnitate (Span® 40), or sorbitan stearate. Other suitable surfactants include, but are not limited to, block copolymers of ethylene oxide and propylene oxide, also known as polyoxyethylene polyoxypropylene block copolymers or polyoxyethylene polypropyleneglycol, such as Poloxamer® 124, Poloxamer® 188, Poloxamer® 237, Poloxamer® 388, or Poloxamer® 407 (BASF Wyandotte Corp.). As described above, a mixture of surfactants can be used in a solid composition of the present invention.

Non-limiting examples of preferred surfactants for the invention include to polysorbate 20, polysorbate 40, polysorbate 60, polysorbate 80, Cremophor RH 40, Cremophor EL, Gelucire 44/14, Gelucire 50/13, D-alpha-tocopheryl polyethylene glycol 1000 succinate (vitamin E TPGS), propylene glycol laurate, sodium lauryl sulfate, and sorbitan monolaurate.

In one embodiment, a solid composition of the present invention comprises an amorphous solid dispersion or a solid solution which includes Compound I (or a pharmaceutically acceptable salt thereof) and a pharmaceutically acceptable hydrophilic polymer. The solid composition also includes a pharmaceutically acceptable surfactant which preferably is formulated in the amorphous solid dispersion or solid solution. The hydrophilic polymer can be selected, for example, from the group consisting of homopolymer of N-vinyl lactam, copolymer of N-vinyl lactam, cellulose ester, cellulose ether, polyalkylene oxide, polyacrylate, polymethacrylate, polyacrylamide, polyvinyl alcohol, vinyl acetate polymer, oligosaccharide, and polysaccharide. As a non-limiting example, the hydrophilic polymer is selected from the group consisting of homopolymer of N-vinyl pyrrolidone, copolymer of N-vinyl pyrrolidone, copolymer of N-vinyl pyrrolidone and vinyl acetate, copolymer of N-vinyl pyrrolidone and vinyl propionate, polyvinylpyrrolidone, methylcellulose, ethylcellulose, hydroxyalkylcelluloses, hydroxypropylcellulose, hydroxyalkylalkylcellulose, hydroxypropylmethylcellulose, cellulose phthalate, cellulose succinate, cellulose acetate phthalate, hydroxypropylmethylcellulose phthalate, hydroxypropylmethylcellulose succinate, hydroxypropylmethylcellulose acetate succinate, polyethylene oxide, polypropylene oxide, copolymer of ethylene oxide and propylene oxide, methacrylic acid/ethyl acrylate copolymer, methacrylic acid/methyl methacrylate copolymer, butyl methacrylate/2-dimethylaminoethyl methacrylate copolymer, poly(hydroxyalkyl acrylate), poly(hydroxyalkyl methacrylate), copolymer of vinyl acetate and crotonic acid, partially hydrolyzed polyvinyl acetate, carrageenan, galactomannan, and xanthan gum. Preferably, the hydrophilic polymer is selected from polyvinylpyrrolidone (PVP) K17, PVP K25, PVP K30, PVP K90, hydroxypropyl methylcellulose (HPMC) E3, HPMC E5, HPMC E6, HPMC E15, HPMC K3, HPMC A4, HPMC A15, HPMC acetate succinate (AS) LF, HPMC AS MF, HPMC AS HF, HPMC AS LG, HPMC AS MG, HPMC AS HG, HPMC phthalate (P) 50, HPMC P 55, Ethocel 4, Ethocel 7, Ethocel 10, Ethocel 14, Ethocel 20, copovidone (vinylpyrrolidone-vinyl acetate copolymer 60/40), polyvinyl acetate, methacrylate/methacrylic acid copolymer (Eudragit) L100-55, Eudragit L100, Eudragit S100, polyethylene glycol (PEG) 400, PEG 600, PEG 1450, PEG 3350, PEG 4000, PEG 6000, PEG 8000, poloxamer 124, poloxamer 188, poloxamer 237, poloxamer 338, or poloxamer 407. More preferably, the hydrophilic polymer is selected from homopolymers of vinylpyrrolidone (e.g., PVP with Fikentscher K values of from 12 to 100, or PVP with Fikentscher K values of from 17 to 30), or copolymers of 30 to 70% by weight of N-vinylpyrrolidone (VP) and 70 to 30% by weight of vinyl acetate (VA) (e.g., a copolymer of 60% by weight VP and 40% by weight VA). The surfactant can be selected, for example, from the group consisting of polyoxyethyleneglycerol triricinoleate or polyoxyl 35 castor oil (Cremophor® EL; BASF Corp.) or polyoxyethyleneglycerol oxystearate, mono fatty acid ester of polyoxyethylene sorbitan, polyoxyethylene alkyl ether, polyoxyethylene alkylaryl ether, polyethylene glycol fatty acid ester, alkylene glycol fatty acid mono ester, sucrose fatty acid ester, and sorbitan fatty acid mono ester. As a non-limited example, the surfactant is selected from the group consisting of polyethylenglycol 40 hydrogenated castor oil (Cremophor® RH 40, also known as polyoxyl 40 hydrogenated castor oil or macrogolglycerol hydroxystearate), polyethylenglycol 60 hydrogenated castor oil (Cremophor® RH 60), a mono fatty acid ester of polyoxyethylene (20) sorbitan (e.g. polyoxyethylene (20) sorbitan monooleate (Tween® 80), polyoxyethylene (20) sorbitan monostearate (Tween® 60), polyoxyethylene (20) sorbitan monopalmitate (Tween® 40), or polyoxyethylene (20) sorbitan monolaurate (Tween® 20)), polyoxyethylene (3) lauryl ether, polyoxyethylene (5) cetyl ether, polyoxyethylene (2) stearyl ether, polyoxyethylene (5) stearyl ether, polyoxyethylene (2) nonylphenyl ether, polyoxyethylene (3) nonylphenyl ether, polyoxyethylene (4) nonylphenyl ether, polyoxyethylene (3) octylphenyl ether, PEG-200 monolaurate, PEG-200 dilaurate, PEG-300 dilaurate, PEG-400 dilaurate, PEG-300 distearate, PEG-300 dioleate, propylene glycol monolaurate, sucrose monostearate, sucrose distearate, sucrose monolaurate, sucrose dilaurate, sorbitan mono laurate, sorbitan monooleate, sorbitan monopalnitate, and sorbitan stearate. Preferably, the surfactant is selected from polysorbate 20, polysorbate 40, polysorbate 60, polysorbate 80, Cremophor RH 40, Cremophor EL, Gelucire 44/14, Gelucire 50/13, D-alpha-tocopheryl polyethylene glycol 1000 succinate (vitamin E TPGS), propylene glycol laurate, sodium lauryl sulfate, or sorbitan monolaurate. More preferably, the surfactant is selected from Tween (e.g., Tween 80, 60, 40 or 20) or D-alpha-tocopheryl polyethylene glycol 1000 succinate. The solid composition may also comprise an amorphous solid dispersion or solid solution of ritonavir, and preferably, ritonavir and Compound I (or a pharmaceutically acceptable salt thereof) are formulated in the same amorphous solid dispersion or solid solution.

In another embodiment, a solid composition of the present invention comprises an amorphous solid dispersion or solid solution which includes Compound I (or a pharmaceutically acceptable salt thereof) and a homopolymer or copolymer of N-vinyl pyrrolidone (e.g., copovidone). The solid composition also comprises a pharmaceutically acceptable surfactant (e.g., vitamin E TPGS, or polysorbate such as polysorbate 80), wherein the surfactant preferably is formulated in the amorphous solid dispersion or solid solution. The solid composition may also comprise an amorphous solid dispersion or solid solution of ritonavir, and preferably, ritonavir and Compound I (or a pharmaceutically acceptable salt thereof) are formulated in the same amorphous solid dispersion or solid solution.

In yet another embodiment, a solid composition of the present invention comprises an amorphous solid dispersion or solid solution which includes Compound I (or a pharmaceutically acceptable salt thereof), copovidone, and a pharmaceutically acceptable surfactant selected from vitamin E TPGS or polysorbate (e.g., polysorbate 80). The amorphous solid dispersion or solid solution may also include another pharmaceutically acceptable surfactant such as propylene glycol laurate (e.g., lauroglycol FCC). The solid composition may comprise an amorphous solid dispersion or solid solution of ritonavir, and preferably, ritonavir and Compound I (or a pharmaceutically acceptable salt thereof) are formulated in the same amorphous solid dispersion or solid solution.

A solid dispersion employed in the present invention preferably comprises or consists of a single-phase (defined in thermodynamics) in which the therapeutic agent (e.g., Compound I and/or ritonavir) and the pharmaceutically acceptable hydrophilic polymer are molecularly dispersed. In such cases, thermal analysis of the solid dispersion using differential scanning calorimetry (DSC) typically shows only one single Tg, and the solid dispersion does not contain any detectable crystalline Compound I or ritonavir as measured by X-ray powder diffraction spectroscopy.

Compound I can be prepared according to the procedures described in WO 2010/030359. Boc-2(S)-amino-non-8-eoic acid dicyclohexylamine salt can be suspended in isopropyl acetate, washed several times with an aqueous citric acid solution and then once with water. The washed product, concentrated and then re-diluted in isopropyl acetate, can be reacted with HCl to produce 2(S)-amino-non-8-eoic acid HCl salt. 5-Methyl-2-pyrazinecarboxylic acid, N,N'-disuccinimidyl carbonate, and N,N-dimethylaminopyridine can be dissolved in N-methyl-2-pyrrolidone (NMP) and stirred. 2(S)-Amino-non-8-eoic acid HCl salt is subsequently added, followed by triethylamine, and stirred to produce (S)-2-(5-methylpyrazine-2-carboxamido)non-8-enoic acid, which can be crystallized out by adding HCl followed by water. (2S,4R)-N-Boc-4-hydroxyproline can be reacted with 6-chlorophenanthridine in NMP, in the presence of sodium t-butoxide, to produce (2S,4R)-1-(tert-butoxycarbonyl)-4-(phenanthridin-6-yloxy)pyrrolidine-2-carboxylic acid. Methyl tertiary butyl ether (MTBE) and water can then be added. The aqueous layer is separated, washed, and then HCl is added, followed by extraction with MTBE. The extracted product can be mixed with diisopropylethylamine (DIPEA) and HATU (CAS # 148893-10-1), and then reacted with (1R,2S)-ethyl-1-amino-2-vinylcyclopropanecarboxylate tosylate salt in dimethylformide (DMF) and toluene. The reaction produces (2S,4R)-tert-butyl 2-((1R,2S)-1-(ethoxycarbonyl)-2-vinylcyclopropylcarbamoyl)-4-(phenanthridin-6-yloxy)pyrrolidine-1-carboxylate, which can be extracted with MTBE and washed with HCl, further extracted, washed, dried, and dissolved in 2-propanol.

HCl can be added to the 2-propanol solution to produce (1R,2S)-ethyl 1-((2S,4R)-4-(phenanthridin-6-yloxy)pyrrolidine-2-carboxamido)-2-vinylcyclopropanecarboxylate, which can be crystallized out by neutralizing with NaOH. (1R,2S)-ethyl 1-((2S,4R)-4-(phenanthridin-6-yloxy)pyrrolidine-2-carboxamido)-2-vinylcyclopropanecarboxylate, (S)-2-(5-methylpyrazine-2-carboxamido)non-8-enoic acid, N-hydroxy-5-norbornene-2,3-dicarboximide, and N-(3-dimethylaminopropyl)-N'-ethylcarbodiimide hydrochloride can be mixed and stirred in DMF, followed by addition of N,N-dimethylethylene diamine. The reaction produces (1R,2S)-ethyl 1-((2S,4R)-1-((S)-2-(5-methylpyrazine-2-carboxamido)non-8-enoyl)-4-(phenanthridin-6-yloxy)pyrrolidine-2-carboxamido)-2-vinylcyclopropanecarboxylate, which can be dissolved in isopropyl acetate and extracted with aqueous $H_3PO_4$, and then extracted with aqueous $K_2HPO_4$. The product can be reacted with di-tert-butyldicarbonate in the presence of dimethylaminopyridine, followed by extraction with a mixture of a citric acid solution and a sodium chloride solution, to produce (1R,2S)-ethyl-1-((2S,4R)-N-(tert-butoxycarbonyl)-1-((S)-2-(5-methylpyrazine-2-carboxamido)non-8-enoyl)-4-(phenanthridin-6-yloxy)pyrrolidine-2-carboxamido)-2-vinylcyclopropanecarboxylate, which can be subject to ring-closing metathesis in the presence of Zhan Catalyst-1B (Zannan Pharma Ltd., Shanghai, China) in toluene to produce (2R,6S,13aS,14aR,16aS,Z)-15-tert-butyl 14a-ethyl 6-(5-methylpyrazine-2-carboxamido)-5,16-dioxo-2-(phenanthridin-6-yloxy)-2,3,5,6,7,8,9,10,11,13a,14,14a,16,16a-tetradecahydrocyclopropa[e]pyrrolo[1,2-a][1,4]diazacyclopentadecine-14a,15(1H)-dicarboxylate. The catalyst can be quenched with imidazole after the reaction.

The ring-closed product in toluene can be solvent switched to acetonitrile, followed by addition of hydrogen chloride in dioxane and heated to produce (2R,6S,3aS,4aR,16aS,Z)-ethyl-6-(5-methylpyrazine-2-carboxamido)-5,16-dioxo-2-(phenanthridin-6-yloxy)-1,2,3,5,6,7,8,9,10,11,13a,14,14a,15,16,16a-hexadecahydrocyclopropa[e]pyrrolo[1,2-a][1,4]diazacyclopentadecine-14a-carboxylate hydrochloride, which can then be isolated, mixed with tetrahydrofuran, water and LiOH·$H_2O$, and then heated and stirred. The reaction mixture can be later cooled, added with aqueous $H_3PO_4$, aqueous NaCl and 2-methyl tetrahydrofuran, and the organic layer is separated, washed and filtered. MeCN is added to the concentrated organic layer, heated and cooled, and then diethylamine is added. The slurry is heated and cooled to form (2R,6S,13aS,14aR,16aS,Z)-6-(5-Methylpyrazine-2-carboxamido)-5,16-dioxo-2-(phenanthridin-6-yloxy)-1,2,3,5,6,7,8,9,10,11,13a,14,14a,15,16,16a-hexadecahydrocyclopropa[e]pyrrolo[1,2-a][1,4]diazacyclopentadecine-14a-carboxylate diethylamine salt, which can be further washed and dried.

The diethylamine salt can be mixed with tetrahydrofuran, 2-methyl tetrahydrofuran and aqueous $H_3PO_4$. The organic layer is separated, washed with aqueous NaCl, and then concentrated and/or purified. The product can be subsequently mixed with NMP, followed by addition of carbonyldiimidazole (CDI) and then 1,8-diazabicyclo[5.4.0]undec-7-ene (DBU). Cyclopropylsulfonamide can be subsequently added. The reaction mixture is stirred for hours. Isopropyl acetate can then be added, followed by aqueous $KH_2PO_4$ and then aqueous $H_3PO_4$. The organic layer can be isolated, washed, and purified to produce Compound I, which can be further dissolved in isopropyl acetate and then the solution is diluted with ethanol. Water can be added to the resulting solution in portionwise manner with adequate hold-times after each addition to ensure de-super-saturation. Water addition is terminated just as the ternary solvent system becomes bi-phasic due to the partial immiscibility of isopropyl acetate, ethanol, water solvent system. The slurry can be stirred for hours and then the solid is isolated via filtration and drying to produce the crystalline hydrate of Compound I.

A solid composition of the present invention can further include one or more other anti-HCV agents. These other anti-HCV agents can be, for example, HCV polymerase inhibitors (including nucleoside or non-nucleoside type of polymerase inhibitors), HCV protease inhibitors, HCV helicase inhibitors, CD81 inhibitors, cyclophilin inhibitors, internal ribosome entry site inhibitors, or HCV NS5A inhibitors. Specific examples of these other anti-HCV agents include, but are not limited to, ribavirin, α-interferon, β-interferon, pegylated interferon-α, pegylated interferon-lambda, telaprevir, boceprevir, ITMN-191, BI-201335, TMC-435, MK-7009, VBY-376, VX-500 (Vertex), PHX-B, ACH-1625, IDX136, IDX316, VX-813 (Vertex), SCH 900518 (Schering-Plough), TMC-435 (Tibotec), ITMN-191

(Intermune, Roche), MK-7009 (Merck), IDX-PI (Novartis), BI-201335 (Boehringer Ingelheim), R7128 (Roche), PSI-7851 (Pharmasset), MK-3281 (Merck), PF-868554 (Pfizer), IDX-184 (Novartis), IDX-375 (Pharmasset), BILB-1941 (Boehringer Ingelheim), GS-9190 (Gilead), BMS-790052 (BMS), and Albuferon (Novartis).

A solid composition of the present invention preferably is a solid oral dosage form. Common solid oral dosage forms suitable for the present invention include, but are not limited to, capsules, dragees, granules, pills, powders and tablets, with capsules and tablets being preferred. A solid oral dosage form of the present invention can also include other excipients or inset diluents, such as sucrose, lactose or starch. Lubricants, coloring agents, releasing agents, coating agents, sweetening or flavoring agents, buffering agents, preservatives, or antioxidants can also be included in a solid oral dosage form of the present invention.

A solid composition of the present invention can be prepared by a variety of techniques such as, without limitation, melt-extrusion, spray-drying, co-precipitation, freeze drying, or other solvent evaporation techniques, with melt-extrusion and spray-drying being preferred. The melt-extrusion process typically comprises the steps of preparing a melt which includes the active ingredient(s), the hydrophilic polymer(s) and preferably the surfactant(s), and then cooling the melt until it solidifies. "Melting" means a transition into a liquid or rubbery state in which it is possible for one component to get embedded, preferably homogeneously embedded, in the other component or components. In many cases, the polymer component(s) will melt and the other components including the active ingredient(s) and surfactant(s) will dissolve in the melt thereby forming a solution. Melting usually involves heating above the softening point of the polymer(s). The preparation of the melt can take place in a variety of ways. The mixing of the components can take place before, during or after the formation of the melt. For example, the components can be mixed first and then melted or be simultaneously mixed and melted. The melt can also be homogenized in order to disperse the active ingredient(s) efficiently. In addition, it may be convenient first to melt the polymer(s) and then to mix in and homogenize the active ingredient(s). In one example, all materials except surfactant(s) are blended and fed into an extruder, while the surfactant(s) is molten externally and pumped in during extrusion.

In another example, the melt comprises Compound I and one or more hydrophilic polymers described above, and the melt temperature is in the range of from 100 to 170 °C, preferably from 120 to 150 °C, and highly preferably from 135 to 140 °C.

In yet another example, the melt comprises Compound I, ritonavir and one or more hydrophilic polymers described above. The melt can also include a pharmaceutically acceptable surfactant described above.

In still another example, the melt comprises Compound I, ritonavir, at least another HCV agent described above, and one or more hydrophilic polymers described above. The melt can also include a pharmaceutically acceptable surfactant described above.

To start a melt-extrusion process, the active ingredient(s) (e.g., Compound I, or a combination of Compound I and ritonavir, or a combination of Compound I, ritonavir and at least another anti-HCV agent) can be employed in their solid forms, such as their respective crystalline forms. The active ingredient(s) can also be employed as a solution or dispersion in a suitable liquid solvent such as alcohols, aliphatic hydrocarbons, esters or, in some cases, liquid carbon dioxide. The solvent can be removed, e.g. evaporated, upon preparation of the melt.

Various additives can also be included in the melt, for example, flow regulators (e.g., colloidal silica), binders, lubricants, fillers, disintegrants, plasticizers, colorants, or stabilizers (e.g., antioxidants, light stabilizers, radical scavengers, and stabilizers against microbial attack).

The melting and/or mixing can take place in an apparatus customary for this purpose. Particularly suitable ones are extruders or kneaders. Suitable extruders include single screw extruders, intermeshing screw extruders or multiscrew extruders, preferably twin screw extruders, which can be corotating or counterrotating and, optionally, be equipped with kneading disks. It will be appreciated that the working temperatures will be determined by the kind of extruder or the kind of configuration within the extruder that is used. Part of the energy needed to melt, mix and dissolve the components in the extruder can be provided by heating elements. However, the friction and shearing of the material in the extruder may also provide a substantial amount of energy to the mixture and aid in the formation of a homogeneous melt of the components.

The melt can range from thin to pasty to viscous. Shaping of the extrudate can be conveniently carried out by a calender with two counter-rotating rollers with mutually matching depressions on their surface. The extrudate can be cooled and allow to solidify. The extrudate can also be cut into pieces, either before (hot-cut) or after solidification (cold-cut).

The solidified extrusion product can be further milled, ground or otherwise reduced to granules. The solidified extrudate, as well as each granule produced, comprises a solid dispersion, preferably a solid solution, of the active ingredient(s) in a matrix comprised of the hydrophilic polymer(s) and optionally the pharmaceutically acceptable surfactant(s). Where the granules do not contain any surfactant, a pharmaceutically acceptable surfactant described above can be added to and blended with the granules. The extrusion product can also be blended with other active ingredient(s) and/or additive(s) before being milled or ground to granules. The granules can be further processed into suitable solid oral dosage forms.

In one example, copovidone and one or more surfactants are mixed and granulated, followed by the addition of aerosil, Compound I and ritonavir. The mixture is then milled. The weight ratio of Compound I over ritonavir can range, for example, from 1:1 to 5:1, such as 1:1, 2:1 or 4:1. For instance, the mixture can contain 10% Compound I and 5% ritonavir by weight. For another instance, the mixture can contain 15% Compound I and 7.5% ritonavir by weight. The mixture is then subject to extrusion, and the extrudate thus produced can be milled and sieved for further processing to make capsules or tablets. Surfactant(s) employed in this example can also be added through liquid dosing during extrusion.

In another example, copovidone and one or more surfactants are mixed and granulated, following by the addition of aerosil and Compound I. The mixture, which may contain for example 15% by weight of Compound I, is then milled and extruded. The extrudate thus produced can be further milled and sieved. Ritonavir extrudate can be similarly prepared. Compound I extrudate can be blended with ritonavir extrudate and then co-compressed to make tablets. Preferably, the weight ratio of Compound I over ritonavir in the blend can range, without limitation, from 1:1 to 1:5, such as 1:1, 2:1 or 4:1.

The approach of solvent evaporation, via spray-drying, provides the advantage of allowing for processability at lower temperatures, if needed, and allows for other modifications to the process in order to further improve powder properties. The spray-dried powder can then be formulated further, if needed, and final drug product is flexible with regards to whether capsule, tablet and/or co-formulation with ritonavir is desired.

Exemplary spray-drying processes and spray-drying equipment are described in K. Masters, SPRAY DRYING HANDBOOK (Halstead Press, New York, 4th ed., 1985). Non-limiting examples of spray-drying devices that are suitable for the present invention include spray dryers manufactured by Niro Inc. or GEA Process Engineering Inc., Buchi Labortechnik AG, and Spray Drying Systems, Inc. A spray-drying process generally involves breaking up a liquid mixture into small droplets and rapidly removing solvent from the droplets in a container (spray drying apparatus) where there is a strong driving force for evaporation of solvent from the droplets. Atomization techniques include, for example, two-fluid or pressure nozzles, or rotary atomizers. The strong driving force for solvent evaporation can be provided, for example, by maintaining the partial pressure of solvent in the spray drying apparatus well below the vapor pressure of the solvent at the temperatures of the drying droplets. This may be accomplished by either (1) maintaining the pressure in the spray drying apparatus at a partial vacuum; (2) mixing the liquid droplets with a warm drying gas (e.g., heated nitrogen); or (3) both.

The temperature and flow rate of the drying gas, as well as the spray dryer design, can be selected so that the droplets are dry enough by the time they reach the wall of the apparatus. This help to ensure that the dried droplets are essentially solid and can form a fine powder and do not stick to the apparatus wall. The spray-dried product can be collected by removing the material manually, pneumatically, mechanically or by other suitable means. The actual length of time to achieve the preferred level of dryness depends on the size of the droplets, the formulation, and spray dryer operation. Following the solidification, the solid powder may stay in the spray drying chamber for additional time (e.g., 5-60 seconds) to further evaporate solvent from the solid powder. The final solvent content in the solid dispersion as it exits the dryer is preferably at a sufficiently low level so as to improve the stability of the final product. For instance, the residual solvent content of the spray-dried powder can be less than 2% by weight. Highly preferably, the residual solvent content is within the limits set forth in the International Conference on Harmonization (ICH) Guidelines. In addition, it may be useful to subject the spray-dried composition to further drying to lower the residual solvent to even lower levels. Methods to further lower solvent levels include, but are not limited to, fluid bed drying, infra-red drying, tumble drying, vacuum drying, and combinations of these and other processes.

Like the solid extrudate described above, the spray dried product contains a solid dispersion, preferably a solid solution, of the active ingredient(s) in a matrix comprised of the hydrophilic polymer(s) and optionally the pharmaceutically acceptable surfactant(s). Where the spray dried product does not contain any surfactant, a pharmaceutically acceptable surfactant described above can be added to and blended with the spray-dried product before further processing.

Before feeding into a spray dryer, the active ingredient(s) (e.g., Compound I, or a combination of Compound I and ritonavir, or a combination of Compound I, ritonavir and at least another anti-HCV agent), the hydrophilic polymer(s), as well as other optional active ingredients or excipients such as the pharmaceutically acceptable surfactant(s), can be dissolved in a solvent. Suitable solvents include, but are not limited to, alkanols (e.g., methanol, ethanol, 1-propanol, 2-propanol or mixtures thereof), acetone, acetone/water, alkanol/water mixtures (e.g., ethanol/water mixtures), or combinations thereof. The solution can also be preheated before being fed into the spray dryer.

The solid dispersion produced by melt-extrusion, spray-drying or other techniques can be prepared into any suitable solid oral dosage forms. In one embodiment, the solid dispersion prepared by melt-extrusion, spray-drying or other techniques (e.g., the extrudate or the spray-dried powder) can be compressed into tablets. The solid dispersion can be either directly compressed, or milled or ground to granules or powders before compression. Compression can be done in a tablet press, such as in a steel die between two moving punches. When a solid composition of the present invention comprises Compound I and ritonavir, or Compound I and another anti-HCV agent, it is possible to separately prepare solid dispersions of each individual active ingredient and then blend the optionally milled or ground solid dispersions before compacting. Compound I and other active ingredient(s) can also be prepared in the same solid dispersion, optionally milled and/or blended with other additives, and then compressed into tablets.

At least one additive selected from flow regulators, disintegrants, bulking agents (fillers) and lubricants may be used in compressing the solid dispersion. These additives can be mixed with ground or milled solid dispersion before compacting. Disintegrants promote a rapid disintegration of the compact in the stomach and keeps the liberated granules separate from one another. Non-limiting examples of suitable disintegrants are cross-linked polymers such as cross-linked polyvinyl pyrrolidone and cross-linked sodium carboxymethylcellulose. Non-limiting examples of suitable bulking agents (also referred to as "fillers") are lactose, calcium hydrogenphosphate, microcrystalline cellulose (e.g., Avicell), silicates, in particular silicium dioxide, magnesium oxide, talc, potato or corn starch, isomalt, or polyvinyl alcohol. Non-limiting examples of suitable flow regulators include highly dispersed silica (e.g., Aerosil), and animal or vegetable fats or waxes. Non-limiting examples of suitable lubricants include polyethylene glycol (e.g., having a molecular weight of from 1000 to 6000), magnesium and calcium stearates, sodium stearyl fumarate, and the like.

Various other additives may also be used in preparing a solid composition of the present invention, for example dyes such as azo dyes, organic or inorganic pigments such as aluminium oxide or titanium dioxide, or dyes of natural origin; stabilizers such as antioxidants, light stabilizers, radical scavengers, stabilizers against microbial attack.

Solid compositions according to certain embodiments of the present invention may contain several layers, for example laminated or multilayer tablets. They can be in open or closed form. "Closed dosage forms" are those in which one layer is completely surrounded by at least one other layer.

In order to facilitate the intake of a solid dosage form, it is advantageous to give the dosage form an appropriate shape. Large tablets that can be swallowed comfortably are therefore preferably elongated rather than round in shape.

A film coat on the tablet further contributes to the ease with which it can be swallowed. A film coat also improves taste and provides an elegant appearance. The film-coat usually includes a polymeric film-forming material such as hydroxypropyl methylcellulose, hydroxypropylcellulose, and acrylate or methacrylate copolymers. Besides a film-forming polymer, the film-coat may further comprise a plasticizer, e.g. polyethylene glycol, a surfactant, e.g. polysorbates, and optionally a pigment, e.g. titanium dioxide or iron oxides. The film-coating may also comprise talc as anti-adhesive. Preferably, the film coat accounts for less than 5 % by weight of a pharmaceutical composition of the present invention.

In another aspect, the present disclosure feature methods of using solid compositions of the present invention to treat HIV infection. The methods comprise administering a solid composition of the present invention to a patient in need thereof. A solid composition of the present invention can be administered either alone, or in combination with one or more other anti-HCV agents, such as those described hereinabove. The specific inhibitory dose for any particular patient will depend upon a variety of factors including the severity of the HCV infection; the activity of Compound I in the particular patient; the specific solid composition employed; the age, body weight, general health, sex and diet of the patient; the time of administration and rate of excretion; the duration of the treatment; drugs used in combination or coincidental with Compound I; and like factors well known in the medical arts.

In one embodiment, a method of the present disclosure comprises administering to a patient in need thereof a solid composition of the present invention and at least another anti-HCV agent, wherein said another anti-HCV agent is selected from HCV polymerase inhibitors (e.g., nucleoside or non-nucleoside HCV polymerase inhibitors), HCV protease inhibitors, HCV helicase inhibitors, CD81 inhibitors, cyclophilin inhibitors, internal ribosome entry site inhibitors, or HCV NS5A inhibitors. Preferably, said another anti-HCV agent is an HCV polymerase inhibitor (e.g., nucleoside or non-nucleoside HCV polymerase inhibitor) or an HCV NS5A inhibitor. The administration of a solid composition of the present invention and another anti-HCV agent(s) can be concurrent or sequential.

The present disclosure also features use of a solid composition of the present invention for the manufacture of medicaments for the treatment of HCV infection.

It should be understood that the above-described embodiments and the following examples are given by way of illustration, not limitation. Various changes and modifications within the scope of the present invention will become apparent to those skilled in the art from the present description.

**Example 1**

Pharmacokinetic (PK) parameters of Compound I and ritonavir were estimated using WinNonlin 5.2 (Pharsight, Mountain View, CA), using non-compartmental analysis. Values below limit of quantification were replaced by zero. Missing values were treated as if they were never drawn. Nominal blood sampling times and doses as specified in the protocol were used for PK analysis.

The following primary pharmacokinetic (PK) parameters were determined for Compound I and ritonavir:

| | |
|---|---|
| $AUC_\infty$ | Area under the concentration versus time curve from time 0 to infinity calculated as $AUC_\infty = AUC_{last} + (C_{last}/K_{el})$, where $C_{last}$ is the last quantifiable concentration |
| Dose-normalized $AUC_\infty$ | Dose-normalized area under the concentration versus time curve from time 0 to infinity ($AUC_\infty$ or $AUC_{(0-Inf)}$:) |

$$AUC_{(0\text{-}Inf)\,norm} = AUC_{(0-Inf)} * \frac{normalized\,dose}{actual\,dose}$$

C$_{max}$           Maximum observed plasma concentration

Dose-normalized C$_{max}$     Dose-normalized maximum observed plasma concentration:

$$C_{max\,norm} = C_{max} * \frac{normalized\,dose}{actual\,dose}$$

T$_{max}$     Time of maximum plasma concentration

**Example 2**

Compound I in crystalline monohydrate and dihydrate forms was mixed with hydrophilic polymers and pharmaceutically acceptable surfactants at various ratios, and dissolved in an organic solvent (acetone or ethanol/water mixtures). The solvent was then removed from the system under heat (75°C) and vacuum, using a Genevac rotary evaporatory or Buchi Rotavap. Solid dispersions of Compound I at various drug loading levels and using different surfactants or polymers were sieved through a 30 mesh screen to reduce particle size. The resultant solid dispersion samples were used for amorphous characterization by X-ray powder diffraction (PXRD), chemical stability, in-vitro dissolution test and dog bioavailability studies.

For dog bioavailability studies, the solid dispersion powder was filled into hard gelatin capsules to achieve target dose of 50 mg. The capsule was co-dosed with a 50 mg of ritonavir. For in-vitro dissolution studies, the release of Compound I was evaluated.

The hydrophilic polymers tested included copovidone, hydroxypropyl methylcellulose acetate succinate (HPMC-AS), and hydroxypropyl methylcellulose grade E5 (HPMC-E5). The surfactants tested included Vitamin E TPGS, polysorbate 20, polysorbate 80, poloxamer, propylene glycol laurate, and span 20. The amount of the surfactant(s) in each solid dispersion tested was no more than 10% by weight, and the amount of Compound I in each solid dispersion ranged from 10 to 40% by weight.

All solid dispersions tested showed that Compound I was in an amorphous form, as indicated by their PXRD patterns. Solid dispersions containing copovidone or HPMC-AS were tested for stability and showed chemical stability after 4 weeks at 40 °C and 75% relative humidity in open dish studies. These solid dispersions also exhibited rapid dissolution rate.

**Example 3**

Two tablet formulations were prepared using spray-drying to produce a solid dispersion powder of amorphous Compound I within a polymer matrix. For the 1$^{st}$ tablet formulation, the spray dried powder contained 17.5% by weight of Compound I, 72.5% by weight of copovidone, and 10% by weight of polysorbate 80. For the 2$^{nd}$ tablet formulation, the spray dried powder contained 17.5% by weight of Compound I, 72.5% by weight of copovidone, 7% by weight of propylene glycol monoloaurate, and 3% by weight of Vitamin E TPGS. For both formulations, acetone was used as a solvent for spray-drying.

The spray dried powder was further dried under vacuum to remove residual solvent. The vacuum dried powder was blended with microcrystalline cellulose, anhydrous dibasic calcium phosphate, pregelatinized starch, croscarmellose sodium, colloidal silicon dioxide, and sodium stearyl fumarate. This blend was optionally dry granulated via roller compaction and then milled to produce granules. The resulting granules were then blended with additional sodium stearyl fumarate prior to being compressed into the final tablet dosage form.

**Example 4**

Compound I and ritonavir were co-extruded using melt-extrusion. Four extrudates were prepared, and then milled and filled into capsules. The 1$^{st}$ extrudate contained Compound I, ritonavir, copovidone, lauroglycol FCC, and Vitamin E TPGS in a weight ration of 10:5:77:5:3 (hereinafter Formulation 1). The 2$^{nd}$ extrudate contained Compound I, ritonavir, copovidone, and polysorbate 80 in a weight ration of 15:7.5:67.5:10 (hereinafter Formulation 2). The 3$^{rd}$ extrudate contained Compound I, ritonavir, copovidone, lauroglycol FCC, and Vitamin E TPGS in a weight ration of 10:5:79:4:2 (hereinafter Formulation 3). The 4$^{th}$ extrudate contained Compound I, ritonavir, copovidone, lauroglycol FCC, and Vitamin E TPGS in a weight ration of 15:7.5:69.5:5:3 (hereinafter Formulation 4). Each of these extrudate capsules contained 50 mg Compound I and 25 mg ritonavir.

Compound I and ritonavir were also separately extruded using melt-extrusion. The Compound I extrudate contained Compound I, copovidone, Lauroglycol FCC, Vitamin E TPGS and aerosol in a weight ratio of 15:76:5:3:1. The ritonavir extrudate contained ritonavir, copovidone, span 20 and aerosol in a weight ratio of 15:74:10:1. Both extrudates were milled, mixed together, and then co-compressed into tablets. Each tablet contained 100 mg Compound I and 50 mg ritonavir (hereinafter Formulation 5).

The bioavailability of the extrudate capsules and the co-compressed tablet was assessed in Beagle dogs after single oral administration. The administered doses were 100 mg Compound I and 50 mg ritonavir per animal. Four dogs (two male and two female dogs) were used in the study. Thirty minutes prior to dosing, each dog received a subcutaneous dose of histamine (100 $\mu$g/kg 0.05 ml/kg in water). See Kahlson et al. J PHYSIOL 174:400-416 (1964); and Akimoto et al. EUR J PHARM BIOPHARM 49:99-102 (2000). Each dogs was subjected to single oral doses of Formulations 1-5 in different weeks, each week with one single dose administration. Plasma samples were collected at 0.33, 1, 2, 4, 6, 8, 12 and 24 hours post-dose administration, and were analyzed for Compound I and ritonavir by LC-MS/MS.

The mean dose-normalized $AUC_\infty$ values of Compound I for Formulations 1-5 were 183.6, 131.6, 188.9, 190.3, and 299.1 $\mu$g·h/ml, respectively, at a 10 mg/kg dose. The mean dose-normalized $C_{max}$ values of Compound I for Formulations 1-5 were 28.5, 24.5, 23.6, 26.8, and 43.3 $\mu$g/ml, respectively, at a 10 mg/kg dose.

The mean dose-normalized $AUC_\infty$ values of ritonavir for Formulations 1-5 were 3.9, 2.8, 2.4, 1.3, and 3.4 $\mu$g·h/ml, respectively, at a 5 mg/kg dose. The mean dose-normalized $C_{max}$ values of ritonavir for Formulations 1-5 were 1.1, 0.8, 0.7, 0.5, and 1.1 $\mu$g/ml, respectively, at a 5 mg/kg dose.

**Claims**

1. A solid composition comprising <u>a solid dispersion which includes</u>

    (1)  (2R,6S,13aS,14aR,16aS,Z)-N-(cyclopropylsulfonyl)-6-(5-methylpyrazine-2-carboxamido)-5,16-dioxo-2-(phenanthridin-6-yloxy)-1,2,3,5,6,7,8,9,10,11,13a,14,14a,15,16,16a-hexadecahydrocyclopropa[e]pyrrolo[1,2-a][1,4]diazacyclopentadecine-14a-carboxamide, or a pharmaceutically acceptable salt thereof, in an amorphous form; <u>and</u>
    (2) a pharmaceutically acceptable hydrophilic polymers;

    <u>said solid composition further comprising</u>

    (3) a pharmaceutically acceptable surfactant.

2. The composition of claim 1, wherein said polymer has a Tg of at least 50 °C.

3. The composition of claim 2, wherein said surfactant has a HLB value of at least 10.

4. The composition of claim 3, further comprising another surfactant having a HLB value of below 10.

5. The composition of claim 2, wherein said solid dispersion is an amorphous solid dispersion which further comprises said surfactant.

6. The composition of claim 2, wherein said polymer is a homopolymer or copolymer of N-vinyl pyrrolidone.

7. The composition of claim 1, wherein said polymer is copovidone.

8. The composition of claim 7, wherein said surfactant is propylene glycol laurate.

9. The composition of claim 8, further comprising D-alpha-tocopheryl polyethylene glycol 1000 succinate.

10. The composition of claim 7, wherein said solid dispersion is an amorphous solid dispersion.

11. The composition of claim 7, where said solid dispersion is a solid solution which comprises said surfactant.

12. The composition according to one of claims 1, 5 or 11, further comprising ritonavir.

13. A process of making the composition of claim 1, comprising drying a solvent in a liquid solution, wherein said solution comprises:

    (1)  (2R,6S,13aS,14aR,16aS,Z)-N-(cyclopropylsulfonyl)-6-(5-methylpyrazine-2-carboxamido)-5,16-dioxo-2-(phenanthridin-6-yloxy)-1,2,3,5,6,7,8,9,10,11,13a,14,14a,15,16,16a-hexadecahydrocyclopropa[e]pyrrolo[1,2-a][1,4]diazacyclopentadecine-14a-carboxamide, or a pharmaceutically acceptable salt thereof;
(2) said polymer; and
(3) said surfactant.

14. A process of making the composition of claim 1, comprising solidifying a melt, wherein said melt comprises:

(1)      (2R,6S,13aS,14aR,16aS,Z)-N-(cyclopropylsulfonyl)-6-(5-methylpyrazine-2-carboxamido)-5,16-dioxo-2-(phenanthridin-6-yloxy)-1,2,3,5,6,7,8,9,10,11,13a,14,14a,15,16,16a-hexadecahydrocyclopropa[e]pyrrolo[1,2-a][1,4]diazacyclopentadecine-14a-carboxamide, or a pharmaceutically acceptable salt thereof;
(2) said polymer; and
(3) said surfactant.

**Patentansprüche**

1. Eine feste Zusammensetzung umfassend eine feste Dispersion, welche folgendes einschließt:

(1)      (2R,6S,13aS,14aR,16aS,Z)-N-(Cyclopropylsulfonyl)-6-(5-methylpyrazin-2-carboxamido)-5,16-dioxo-2-(phenanthridin-6-yloxy)-1,2,3,5,6,7,8,9,10,11,13a,14,14,a,15,16,16a-hexadecahydrocyclopropa[e]pyrrolo[1,2-a] [1,4]diazacyclopentadecin-14a-carboxamid oder ein pharmazeutisch verträgliches Salz davon, in einer amorphen Form; und
(2) ein pharmazeutisch verträgliches hydrophiles Polymer; wobei die feste Zusammensetzung weiter
(3) einen pharmazeutisch verträglichen oberflächenaktiven Stoff umfasst.

2. Die Zusammensetzung gemäß Anspruch 1, worin das Polymer eine $T_g$ von mindestens 50°C hat.

3. Die Zusammensetzung gemäß Anspruch 2, worin der oberflächenaktive Stoff einen HLB-Wert von mindestens 10 hat.

4. Die Zusammensetzung gemäß Anspruch 3, weiter umfassend einen anderen oberflächenaktiven Stoff mit einem HLB-Wert von unter 10.

5. Die Zusammensetzung gemäß Anspruch 2, worin die feste Dispersion eine amorphe feste Dispersion ist, welche weiter den oberflächenaktiven Stoff umfasst.

6. Die Zusammensetzung gemäß Anspruch 2, worin das Polymer ein Homopolymer oder Copolymer von N-Vinylpyrrolidon ist.

7. Die Zusammensetzung gemäß Anspruch 1, worin das Polymer Copovidon ist.

8. Die Zusammensetzung gemäß Anspruch 7, worin der oberflächenaktive Stoff Propylenglykollaurat ist.

9. Die Zusammensetzung gemäß Anspruch 8, weiter umfassend D-alpha-Tocopherylpolyethylenglykol 1000 Succinat.

10. Die Zusammensetzung gemäß Anspruch 7, worin die feste Dispersion eine amorphe feste Dispersion ist.

11. Die Zusammensetzung gemäß Anspruch 7, worin die feste Dispersion eine feste Lösung ist, welche den oberflächenaktiven Stoff umfasst.

12. Die Zusammensetzung gemäß einem der Ansprüche 1, 5 oder 11, weiter umfassend Ritonavir.

13. Ein Verfahren zur Herstellung der Zusammensetzung gemäß Anspruch 1, umfassend das Trocknen eines Lösungsmittels in einer flüssigen Lösung, worin die Lösung folgendes umfasst:

(1)      (2R,6S,13aS,14aR,16aS,Z)-N-(Cyclopropylsulfonyl)-6-(5-methylpyrazin-2-carboxamido)-5,16-dioxo-2-(phenanthridin-6-yloxy)-1,2,3,5,6,7,8,9,10,11,13a,14,14a,15,16,16a-hexadecahydrocyclopropa[e]pyrrolo[1,2-a] [1,4]diazacyclopentadecin-14a-carboxamid oder ein pharmazeutisch verträgliches Salz davon;

(2) das Polymer, und

(3) den oberflächenaktiven Stoff.

14. Ein Verfahren zur Herstellung der Zusammensetzung gemäß Anspruch 1, umfassend das Festwerdenlassen einer Schmelze, worin die Schmelze folgendes umfasst:

(1)    (2R,6S,13aS,14aR,16aS,Z)-N-(Cyclopropylsulfonyl)-6-(5-methylpyrazin-2-carboxamido)-5,16-dioxo-2-(phenanthridin-6-yloxy)-1,2,3,5,6,7,8,9,10,11,13a,14,14a,15,16,16a-hexadecahydrocyclopropa[e]pyrrolo[1,2-a] [1,4]diazacyclopentadecin-14a-carboxamid, oder ein pharmazeutisch verträgliches Salz davon;

(2) das Polymer, und

(3) den oberflächenaktiven Stoff.

**Revendications**

1. Composition solide comprenant une dispersion solide qui inclut

(1)  du  (2R,6S,13aS,14aR,16aS,Z)-N-(cyclopropylsulfonyl)-6-(5-méthylpyrazine-2-carboxamido)-5,16-dioxo-2-(phénanthridin-6-yloxy)-1,2,3,5,6,7,8,9,10,11,13a,14,14a,15,16,16a-hexadécahydrocyclopropa[e]pyrrolo[,2-a][1,4]diazacyclopentadécine-14a-carboxamide, ou un sel pharmaceutiquement acceptable de celui-ci, sous une forme amorphe ; et

(2) un polymère hydrophile pharmaceutiquement acceptable ; ladite composition solide comprenant en outre

(3) un tensioactif pharmaceutiquement acceptable.

2. Composition selon la revendication 1, dans laquelle ledit polymère a une Tg d'au moins 50 °C.

3. Composition selon la revendication 2, dans laquelle ledit tensioactif a un rapport hydro-lipophile d'au moins 10.

4. Composition selon la revendication 3 comprenant en outre un autre tensioactif ayant un rapport hydro-lipophile inférieur à 10.

5. Composition selon la revendication 2, dans laquelle ladite dispersion solide est une dispersion solide amorphe qui comprend en outre ledit tensioactif.

6. Composition selon la revendication 2, dans laquelle ledit polymère est un homopolymère ou un copolymère de N-vinylpyrrolidone.

7. Composition selon la revendication 1, dans laquelle ledit polymère est la copovidone.

8. Composition selon la revendication 7, dans laquelle ledit tensioactif est le laurate de propylène glycol.

9. Composition selon la revendication 8, comprenant en outre du succinate de D-alpha-tocophéryl polyéthylène glycol 1000.

10. Composition selon la revendication 7, dans laquelle ladite dispersion solide est une dispersion solide amorphe.

11. Composition selon la revendication 7, où ladite dispersion solide est une solution solide qui comprend ledit tensioactif.

12. Composition selon l'une quelconque des revendications 1, 5 ou 11, comprenant en outre du ritonavir.

13. Procédé de préparation de la composition selon la revendication 1, comprenant le séchage d'un solvant dans une solution liquide, dans lequel ladite solution comprend :

(1)  du  (2R,6S,13aS,14aR,16aS,Z)-N-(cyclopropylsulfonyl)-6-(5-méthylpyrazine-2-carboxamido)-5,16-dioxo-2-(phenanthridin-6-yloxy)-1,2,3,5,6,7,8,9,10,11,13a,14,14a,15,16,16a-hexadécahydrocyclopropa[e]pyrrolo[1,2-a][1,4]diazacyclopentadécine-14a-carboxamlde, ou un sel pharmaceutiquement acceptable de celui-ci ;

(2) ledit polymère ; et

(3) ledit tensioactif.

**14.** Procédé de préparation de la composition selon la revendication 1, comprenant la solidification d'une masse fondue, dans lequel ladite masse fondue comprend :

(1) du (2R,6S,13aS,14aR,16aS,Z)-N-(cyclopropylsulfonyl)-6-(5-méthylpyrazine-2-carboxamido)-5,16-dioxo-2-(phénanthridin-6-yloxy)-1,2,3,5,6,7,8,9,10,11,13a,14,14a,15,16,16a-hexadécahydrocyclopropa[e]pyrrolo[1,2-a][1,4]diazacyclopentadécine-14a-carboxamide, ou un sel pharmaceutiquement acceptable de celui-ci ;
(2) ledit polymère ; et
(3) ledit tensioactif.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2010030359 A

**Non-patent literature cited in the description**

- INTRODUCTION TO PHYSICAL POLYMER SCIENCE. John Wiley & Sons, Inc, 1992
- POLYMER HANDBOOK. John Wiley & Sons, Inc, 1975
- **FIEDLER, H.B.** ENCYLOPEDIA OF EXCIPIENTS. ECV-Editio-Cantor-Verlag, 2002
- **KAHLSON et al.** *J PHYSIOL,* 1964, vol. 174, 400-416
- **AKIMOTO et al.** *EUR J PHARM BIOPHARM,* 2000, vol. 49, 99-102